# EUROPEAN PATENT SPECIFICATION

(11) **EP 2 505 195 B1**
(45) Date of publication and mention of the grant of the patent: **30.10.2013**
(21) Application number: 12162459.7
(22) Date of filing: 30.03.2012
(51) Int. Cl.: A61K 31/10, A61K 31/192, A61K 9/00

(54) **Topical pharmaceutical compositions of ketoprofen and methylsulfonylmethane**
Topische pharmazeutische Zusammensetzungen aus Ketoprofen und Methylsuflonylmethan
Compositions pharmaceutiques topiques de kétoprofène et de méthylsulfonylméthane

(30) Priority: 01.04.2011 TR 201103183
(43) Date of publication of application: 03.10.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Önder, Ramazan, 34398 Istanbul (TR)
(74) Representative: Sevinç, Erkan

(56) References cited:
- US-A1- 2003 235 610
- US-A1- 2005 232 980
- US-A1- 2010 063 152
- US-B1- 6 399 093
- US-B1- 6 444 234

## Description

### Field of Invention

The present invention relates to a topical pharmaceutical composition of ketoprofen and methylsulfonylmethane comprising at least one penetration enhancer and one or more gelling agent. Furthermore, the invention relates to process for preparing the said topical pharmaceutical composition and its use for the treatment of pain and inflammatory symptoms associated with muscle-skeletal system, osteoarthritis and rheumatoid arthritis.

### Background of Invention

Ketoprofen is a well known, propionic acid derivative, also known as NSAID (non-steroidal anti-inflammatory drug), with the analgesic and anti-inflammatory activities it possesses. It is used in rheumatoid arthritis, osteoarthritis, ankylosing spondylitis, acute articular and periarticular disorders, cervical spondylitis, low back pain, painful musculoskeletal conditions, acute gout, dysmenorrhoea and control of pain and inflammation following orthopaedic surgery. Its chemical structure is shown with Formula I given below.

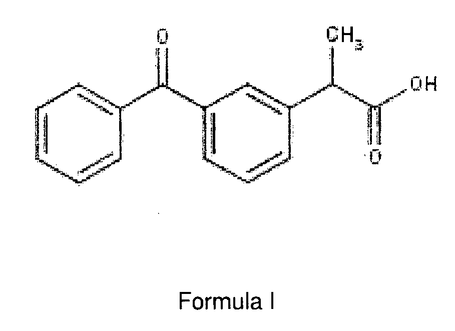

Ketoprofen is mostly administrated orally. One disadvantage of the oral administration of ketoprofen comprising compositions is that the patient is likely to experience unpleasant side effects, including gastrointestinal irritation. While such irritation may also result in chronic stomach upset, in some cases this can quickly manifest itself in spontaneous gastric bleeding, which can be life threatening.

Thus, the use of ketoprofen in treating local pains and inflammations may cause a problem especially for those who have gastrointestinal system disorders. It is possible to develop various locally-administrable topical forms of ketoprofen, in order to avoid the systemic side-effects thereof. The skin absorption rate of the relevant product to be used in topical applications, however, is quite significant. Enhancing the absorption rate provides ease of application and increases the molecule's efficiency.

Methylsulfonylmethane (MSM) is an organosulfur compound and is also known as dimethyl sulfone (DMSO2). It occurs naturally in food in a variety of fruits, vegetables and grains. MSM is commonly used for osteoarthritis, seasonal allergic rhinitis, interstitial cystitis and snoring. MSM is the primary oxidative metabolite product of dimethyl sulfoxide (DMSO).

An additional problem associated with oral pharmaceutical compositions, is that the concentration levels which must be achieved in the bloodstream must be significant in order to effectively treat distal areas of pain and inflammation. These levels are often much higher than would be necessary if it were possible to more accurately target the particular site of pain and injury. Thus there exists a need for a transdermal analgesic formulation which is capable of distal application and which has the ability to alleviate pain and inflammation in a local way.

Also in acute disorders, there arises the need of enhancing the absorption rate at the site of administration. For instance, during which local pains associated with injuries in sportive events are to be urgently alleviated, it becomes necessary to apply local anesthesia to the relevant site.

U.S. Pat. No. 6,444,234 discloses a liquid carrier composition effective for the transdermal delivery of a medicament having a given polarity, said formulation comprising (a) at least one non-aqueous non-toxic solvent; (b) limonene, lemon oil or mixture of limonene and lemon oil; (c) methylsulfonylmethane; (d) a skin stabilizer (e) a solute modifier; and (f) adenosine triphosphate (ATP) or a compound which induces generation of cyclic adenosine 3'5'monophosphate cAMP in situ or cyclic guanosine monophosphate (cGMP) in situ.

In prior art, there are also several patents which disclose NSAIDs with methylsulfonylmethane mostly in oral pharmaceutical dosage forms but none of them selected particularly ketoprofen in a topical formulation to combine with methylsulfonlymethane because of its poor transdermal absorption properties.

U.S. Pat. No. 6,416,772 discloses a liquid composition applied transdermally for relief of pain comprising alcohol, glycerin and an analgesic agent; the analgesic agent comprising a derivative of salicylic acid, methylsulfonylmethane and emu oil. But it is silent about the penetration problems and the use of dimethyl sulfoxide and gelling agents in combination with methylsulfonylmethane and ketoprofen.

U.S. Pat. No. 6,399,093 discloses a method and composition for the treatment of musculoskeletal disorders in mammals by the application of a topical composition comprising a permeation enhancing amount of one or more penetration enhancers, and one or more bioaffecting agents to provide anti-inflammatory relief and analgesia to the applied body part. Said penetration enhancers are a group consisting of alcohols, polyols, sulfoxides, esters, ketones, amides, oleates, surfactants, alkanoic acids, lactam compounds, alkalols, dialkylamino acetates. However, it is silent about the use of dimethyl sulfoxide and gelling agents in combination with methylsulfonylmethane and ketoprofen.

U.S. Pat. No. 2010/0063152 A1 relates to the treatment of edema, more particularly methods and formulations provided for the treatment of localized edema. A metal ion sequestrant is topically administered to a subject afflicted with localized edema in combination with a permeation enhancer selected from methylsulfonylmethane and a combination of methylsulfonylmethane and dimethylsulfoxide. However, the invention does not select ketoprofen and the gelling agents in combination with MSM and DMSO and silent about the related problems.

The compositions described above have at least one disadvantage in that the amount of drug delivered transdermally is not maximized. Accordingly, there exists need for a percutaneous delivery system for the drug ketoprofen and methylsulfonylmethane which optimizes the delivery of them through the skin.

It is therefore an object of the present invention to provide a topical pharmaceutical composition of ketoprofen and methylsulfonylmethane which is more effective for purposes of percutaneous delivery of them through the skin.

### Summary of the Invention

The present invention relates to an easily applicable ketoprofen and methylsulfonylmethane topical pharmaceutical composition as defined in claim 1, which overcomes the above described problems in prior art and have additive advantages over them.

Accordingly, the main object of the present invention is to increase the rate of percutaneous penetration, thereby shortening the time period in which the active agents exert their effect.

The rate of percutaneous penetration of said combination is enhanced with the dimethyl sulfoxide and gelling agents it contains. Also surface active agents has a synergistic effect over this penetration enhancing activity of them.

Another object of the present invention is to obtain a stable topical pharmaceutical composition of ketoprofen and methylsulfonylmethane during the shelf-life and to exhibit high safety when applied to skin.

A further object of the present invention is to obtain a formulation with local anesthetic effect, with the menthol used in said combination stimulating the receptors by which the cold sensation is perceived.

Accordingly, a topical pharmaceutical composition, more specifically a gel composition has been developed to achieve all objects referred above.

In a preferred embodiment according to the present invention, said novelty is realized with ketoprofen and methylsulfonylmethane comprising at least one penetration enhancer and one or more gelling agent.

According to the preferred embodiment, the penetration enhancer is dimethyl sulfoxide.

In another preferred embodiment according to the present invention, the gelling agents are selected from the group consisting hydroxypropyl cellulose, hydroxypropyl methyl cellulose, methyl cellulose, carboxymethyl cellulose, carbomer, carbomer copolymers, aluminum monostearat, dextrin, pectin, carrageanenor mixtures thereof. Preferably the gelling agents are hydroxypropyl cellulose and carbomer.

According to the preferred embodiment, the weight ratio of hydroxypropyl cellulose and carbomer is from 20:1 to 1:10 by weight, preferably it is from 5:1 to 1:5 by weight of the total composition.

According to the preferred embodiment of the present invention the amount of dimethyl sulfoxide is from 0.5 to 30.0% by weight of the total composition, preferably it is 5.0 to 25.0% by weight of the total composition.

According to a preferred embodiment of the present invention, the topical pharmaceutical composition, further comprises surface active agents which are selected from the group comprising polysorbate, glyceryl monostearate, polyethylene glycol succinate, oleic acid, dietanolamin, sodium lauryl sulfate, propylene glycol or mixtures thereof; preferably the surface active agent is polysorbate.

According to the preferred embodiment of the present invention the amount of polysorbate is 0.05 to 5.0% by weight of the total composition, preferably it is 0.10 to 3.0% by weight of the total composition.

According to a preferred embodiment of the present invention, the topical pharmaceutical composition further comprises menthol, wherein the amount of menthol is between 0.10 to 15.0% by weight of the total composition, preferably it is 1.0 to 10.0% by weight of the total composition.

According to a preferred embodiment of the present invention, the topical pharmaceutical composition, further comprises viscosity enhancers, dissolving solvents, preservatives, antioxidants or mixtures thereof.

According to a preferred embodiment of the present invention, the topical pharmaceutical composition is in the form of gel, ointment, cream, spray or lotion, preferably it is in the form of gel.

In a further preferred embodiment of the present invention, said topical pharmaceutical composition comprise the following;

| | | |
|---|---|---|
| a. | ketoprofen | 0.50 to 15.0 % by weight |
| b. | methylsulfonylmethane | 1.0 to 20.0 % by weight |
| c. | hydroxypropyl cellulose | 0.05 to 10.0 % by weight |
| d. | carbomer | 0.01 to 5.0 % by weight |
| e. | dimethyl sulfoxide | 0.5 to 30.0 % by weight |
| f. | polyethylene glycol | 1.0 to 50.0 % by weight |
| g. | menthol | 0.10 to 15.0 % by weight |
| h. | polysorbate | 0.05 to 5.0 % by weight |
| i. | propyl paraben | 0.001 to 2.0 % by weight |
| j. | methyl paraben | 0.01 to 2.0 % by weight |
| k. | butylated hydroxytoluene | 0.001 to 0.10 % by weight |
| l. | glycerin | 1.0 to 50.0 % by weight |
| m. | purified water | 1.0 to 50.0 % by weight |
| n. | ethyl alcohol | 1.0 to 75.0 % by weight |
| o. | NaOH/HCl | pH (5.5 ±1.0) |

Another embodiment of the present invention provides a method for preparing the topical pharmaceutical composition according to the present invention and this method comprising the steps of;
a. adding carbomer, glycerin, polyethylene glycol, dimethyl sulfoxide into purified water and swelling this mixture under stirring for 60 min. and homojenizing so as to yield the first mixture,
b. adding NaOH or HCl to the first mixture to adjust the pH and stirring,
c. adding ethyl alcohol to another container and adding ketoprofen, methylsulfonylmethane, menthol, polysorbate, methyl paraben, propyl paraben, butylated hydroxytoluene and then adding hydroxypropyl cellulose into this mixture and stirring until they are dissolved for about 90 min., homojenizing for about 5 min. so as to give the second mixture,
d. adding the second mixture into the first mixture under stirring and then filling up the volume with ethyl alcohol and stirring 10 more min.
e. bringing into a gelled state and continuing by filling step.

According to another preferred embodiment of the present invention, the topical pharmaceutical composition is used in the treatment of pain and inflammatory symptoms associated with muscle-skeletal system, osteoarthritis and rheumatoid arthritis.

Further advantages and embodiments of the present invention will become apparent from the following description

### Detailed Description of Invention

According to the present invention, a novel formulation with anti-inflammatory and analgesic activities is obtained, which is surprisingly rapidly absorbed and gives local anesthetic effect.

The topical pharmaceutical compositions of the invention comprise from 0.5 to 15.0% ketoprofen, preferably from 1.0 to 5.0% by weight of the total composition and comprise from 1.0 to 20.0% methylsulfonylmethane, preferably from 5.0 to 15.0% by weight of the total composition.

The topical pharmaceutical compositions of the invention comprise from 0.5 to 30.0% dimethyl sulfoxide, preferably from 5.0 to 25.0%, more preferably from 10.0 to 20.0% by weight of the total composition. Dimethyl sulfoxide helps the composition of the present invention to improve and enhance the penetrating and spreading properties through the skin. It also helps to carry out other components easily and without damaging the membranes into biological system. These properties, surprisingly is found to have synergistic effect over ketoprofen and methylsulfonlymethane topical composition to have better percutaneous penetration. Accordingly, dimethyl sulfoxide is used as a topical analgesic, a vehicle for topical application of pharmaceuticals, as an anti-inflammatory and an antioxidant. Other suitable penetration enhancers which can be used for the composition of the present invention may comprise 1,3-didocyl urea, 1,3-difenyl urea, 3-caren, 7-oxabicylo 2,2-heptan, ascaridol, dimetyl isosorbide, dimetyl formamide (DMF), d-Limonen, isopropyl myristat, carveol, carvon, menton, N-metyl-2-pyrolidon, NN-dimetyl toluamide, oleic acid, pinen oxide, puiegon, scylohexan oxide, siklopenten oxide, sodyum lauryl sulfate, terpinen-4-oi urea, a-pinen, a-terpineol or mixtures thereof.

The topical pharmaceutical compositions of the invention comprise from 0.01 to 15.0% gelling agents, preferably from 0.05 to 10.0% by weight. Suitable gelling agents consist of hydroxypropyl cellulose, hydroxypropyl methyl cellulose, methyl cellulose, carboxymethyl cellulose, carbomer, carbomer copolymers, aluminum monostearat, dextrin, pectin, carrageanen or mixtures thereof. Preferably the gelling agents are hydroxypropyl cellulose and carbomer. Surprisingly it is found that when the weight ratio of hydroxypropyl cellulose to carbomer is from 20:1 to 1:10 by weight, preferably from 5:1 to 1:5 by weight; it enhance the penetration properties of the formulation in combination with dimethyl sulfoxide. Accordingly, hydroxypropyl cellulose and carbomer help to obtain the desired viscosity in a stable level to enhance the penetration of the topical composition and their stabilizer and emulsifier property is also help them to show this effect easily.

Suitable surface active agents may comprise but not limited to polysorbate, glyceryl monostearat, polyethylene glycol succinate, oleic acid, diethanolamine, sodium lauril sulfate, propylene glycol or mixtures thereof. Preferably the surface active agent is polysorbate 20, polysorbate 40, polysorbate 60, polysorbate 80 or mixtures thereof. The most preferred one is polysorbate 80. The amount of polysorbate 80 is from 0.05 to 5.0%, preferably 0.1 to 3.0% by weight of the total composition. Polysorbate has also a stabilisator effect when it is used in these amounts and helps the formulation to be stable over the shelf life.

Furthermore, the topical pharmaceutical compositions of the invention comprise menthol from 0.10 to 15.0%, preferably from 1.0 to 10.0% by weight of the total composition. Menthol used in the formulation gives anesthetic effect at the side of administration as a result of stimulating the receptors by which cold sensation is perceived.

Methylsulfonylmethane and menthol provide significant relief of pain associated with mild to moderate muscle strain in adult patients. Also menthol helps to mask the bad odour of the active ingredients and other excipients used in the formulation to obtain a good patient compliance when applying to skin.

The pharmaceutical compositions according to the present invention may also comprise one or more pharmaceutically acceptable excipients. Such proper pharmaceutically acceptable excipients comprise, but are not limited to viscosity enhancers, dissolving solvents, preservatives, antioxidants or mixtures thereof.

Suitable viscosity enhancers may comprise but not limited to glycerin, pullulan, dextran, cellulose and derivatives, chitosan, carbomer or mixtures thereof. Preferably the viscosity enhancer is glycerin and the amount is from 1.0 to 50.0%, more preferably from 5.0 to 30.0% by weight of the total composition. These amounts of glycerin improve the spreading properties and minimize any balling up or drying of the compositions of the present invention when it is rubbed on the skin.

Suitable dissolving solvents may comprise but not limited to ethyl alcohol, polyethylene glycol, glycerin, isopropyl alcohol and purified water. Preferably ethyl alcohol, polyethylene glycol and purified water are used. Ethyl alcohol is also utilized as a microbiological preservative.

Suitable preservatives may comprise but not limited to methylparaben, propylparaben, sodium and potassium benzoate, imidurea, monothioglicol, phenyl mercuric derivatives, sodium sulfate, sodium meta bisulfate, potassium sorbate or mixtures thereof. Preferbly the preservatives are methylparaben and propylparaben. The amount of the preservatives is from 0.001 to 3.0%, more preferably 0.001 to 2.0% by weight of the total composition.

Suitable antioxidants may comprise but not limited to butylated hydroxyl toluene, butyl hydroxy anisole, alpha tocopherol, monothioglicol, sodium meta bisulfate, potassium meta bisulfate or mixtures thereof. Preferably the antioxidant is butylated hydroxyl toluene, The amount of the antioxidants is from 0.001 to 1.0%, preferably from 0.001 to 0.50% by weight of the total composition.

The pharmaceutical compositions according to the present invention provide spreadable, semi-solid and jelly-like gel compositions of ketoprofen and methylsulfonylmethane. However, the topical compositions of the invention may also take the form of ointment, cream, spray or lotion.

Accordingly, the present invention may be used for treating pain and inflammatory symptoms associated with muscle-skeletal system, osteoarthritis and rheumatoid arthritis.

### Example

| **Content** | **amount (%) (w/w)** |
|---|---|
| Ketoprofen | 0.50 to 15.0 |
| Methylsulfonylmethane | 1.0 to 20.0 |
| Hydroxypropyl cellulose | 0.05 to 10.0 |
| Carbomer | 0.01 to 5.0 |
| Dimethyl sulfoxide | 0.50 to 30.0 |
| Polyethylene glycol | 1.0 to 50.0 |
| Menthol | 0.10 to 15.0 |
| Polysorbate 80 | 0.05 to 5.0 |
| Propyl paraben | 0.001 to 2.0 |
| Methyl paraben | 0.01 to 2.0 |
| Butylated hydroxytoluene | 0.01 to 0.10 |
| Glycerin | 1.0 to 50.0 |
| Purified water | 1.0 to 50.0 |
| Ethyl alcohol | 1.0 to 75.0 |
| NaOH/HCL | pH 5.5 ±1.0 |

Carbomer, glycerin, polyethylene glycol and dimethyl sulfoxide is added into purified water under stirring and the mixture is swollen by keeping it stirred for about 60 min and homogenised. Thus, the first mixture is obtained, and the pH is adjusted with NaOH or HCL. Ketoprofen, methylsulfonylmethane, menthol, polysorbate 80, methyl paraben, propyl paraben and butylated hydroxytoluene is dissolved in a separate container in ethyl alcohol. Thus, the second mixture is obtained. Then hydroxypropyl cellulose is added into the second mixture under stirring for about 90 min and then homogenized for 5 more min. The second mixture is added to the first mixture under stirring for about 10 min and filled up with ethyl alcohol. Then, it is brought into a gelled state and the procedure is completed and continue by filling step.

## Claims

1. A topical pharmaceutical composition of ketoprofen and methylsulfonylmethane comprising at least one penetration enhancer and gelling agents, wherein the penetration enhancer is dimethyl sulfoxide and the gelling agents are hydroxypropyl cellulose and carbomer, and wherein the weight ratio of hydroxypropyl cellulose to carbomer is from 20:1 to 1:10.

2. The topical pharmaceutical composition according to claim 1, wherein the weight ratio of hydroxypropyl cellulose to carbomer is from 5:1 to 1:5 by weight of the total composition.

3. The topical pharmaceutical composition according to claim 1, wherein the amount of dimethyl sulfoxide is from 0.50 to 30.0 % by weight of the total composition, preferably it is 5.0 to 25.0 % by weight of the total composition.

4. The topical pharmaceutical composition according to any of the preceding claims, further comprising surface active agents.

5. The topical pharmaceutical composition according to claim 4, wherein the surface active agent is selected form the group comprising, polysorbate, glyceryl monostearate, polyethylene glycol succinate, oleic acid, dietanolamin, sodium lauryl sulfate, propylene glycol or mixtures thereof; preferably the surface active agent is polysorbate.

6. The topical pharmaceutical composition according to claim 5, wherein the amount of polysorbate is 0.05 to 5.0 % by weight of the total composition, preferably it is 0.10 to 3.0 % by weight of the total composition.

7. The topical pharmaceutical composition according to any of the preceding claims, further comprising menthol.

8. The topical pharmaceutical composition according to claim 7, wherein the amount of menthol is between 0.10 to 15.0 % by weight of the total composition, preferably it is 1.0 to 10.0 % by weight of the total composition.

9. The topical pharmaceutical composition according to any of the preceding claims, further comprising viscosity enhancers, dissolving solvents, preservatives, antioxidants or mixtures thereof.

10. The topical pharmaceutical composition according to any of the preceding claims, wherein it is in the form of gel, ointment, cream, spray or lotion, preferably it is in the form of gel.

11. The topical pharmaceutical composition according to any of the preceding claims, comprising,
| | | |
|---|---|---|
| a. | ketoprofen | 0.50 to 15.0 % by weight |
| b. | methylsulfonylmethane | 1.0 to 20.0 % by weight |
| c. | hydroxypropyl cellulose | 0.05 to 10.0 % by weight |
| d. | carbomer | 0.01 to 5.0 % by weight |
| e. | dimethyl sulfoxide | 0.5 to 30.0 % by weight |
| f. | polyethylene glycol | 1.0 to 50.0 % by weight |
| g. | menthol | 0.10 to 15.0 % by weight |
| h. | polysorbate | 0.05 to 5.0 % by weight |
| i. | propyl paraben | 0.001 to 2.0 % by weight |
| j. | methyl paraben | 0.01 to 2.0 % by weight |
| k. | butylated hydroxytoluene | 0.001 to 0.10 % by weight |
| l. | glycerin | 1.0 to 50.0 % by weight |
| m. | purified water | 1.0 to 50.0 % by weight |
| n. | ethyl alcohol | 1.0 to 75.0 % by weight |
| o. | NaOH or HCl | pH (5.5 ±1.0) |

12. Process for preparing the topical pharmaceutical composition according to any of the preceding claims, comprising the steps of;
a. adding carbomer, glycerin, polyethylene glycol, dimethyl sulfoxide into purified water and swelling this mixture under stirring for 60 min. and homojenizing so as to yield the first mixture,
b. adding NaOH or HCI to the first mixture to adjust the pH and stirring,
c. adding ethyl alcohol to another container and adding ketoprofen, methylsulfonylmethane, menthol, polysorbate, methyl paraben, propyl paraben, butylated hydroxytoluene and then adding hydroxypropyl cellulose into this mixture and stirring until they are dissolved for about 90 min., homojenizing for about 5 min. so as to give the second mixture,
**d**. adding the second mixture into the first mixture under stirring and then filling up the volume with ethyl alcohol and stirring 10 more min.
**e**. bringing into a gelled state and continuing by filling step.

13. The topical pharmaceutical composition according to any previous claims, for use in the treatment of pain and inflammatory symptoms associated with muscle-skeletal system, osteoarthritis and rheumatoid arthritis.

## Patentansprüche

1. Topische pharmazeutische Zusammensetzung aus Ketoprofen und Methylsulfonylmethan umfassend zumindest einen Penetrationsförderer und Geliermittel, wobei es sich bei dem Penetrationsförderer um Dimethylsulfoxid und bei den Geliermitteln um Hydroxypropylcellulose und Carbomer handelt, und wobei das Gewichtsverhältnis von Hydroxypropylcellulose zu Carbomer zwischen 20:1 und 1:10 beträgt.

2. Topische pharmazeutische Zusammensetzung nach Anspruch 1, bei der das Gewichtsverhältnis von Hydroxypropylcellulose zu Carbomer, auf das Gewicht der Gesamtzusammensetzung bezogen, zwischen 5:1 und 1:5 beträgt.

3. Topische pharmazeutische Zusammensetzung nach Anspruch 1, bei der die Dimethylsulfoxidmenge zwischen 0,50 und 30,0 Gew.-% der Gesamtzusammensetzung, vorzugsweise 5,0 bis 25,0 Gew.-% der Gesamtzusammensetzung, beträgt.

4. Topische pharmazeutische Zusammensetzung nach einem der voranstehenden Ansprüche, des Weiteren umfassend Tenside.

5. Topische pharmazeutische Zusammensetzung nach Anspruch 4, bei der das Tensid ausgewählt ist aus der Gruppe umfassend Polysorbat, Glycerylmonostearat, Polyethylenglykolsuccinat, Oleinsäure, Dietanolamin, Natriumlaurylsulfat, Propylenglykol oder Mischungen daraus; vorzugsweise handelt es sich bei dem Tensid um Polysorbat.

6. Topische pharmazeutische Zusammensetzung nach Anspruch 5, bei der die Polysorbatmenge 0,05 bis 5,0 Gew.-% der Gesamtzusammensetzung beträgt, vorzugsweise bei 0,10 bis 3,0 Gew.-% der Gesamtzusammensetzung liegt.

7. Topische pharmazeutische Zusammensetzung nach einem der voranstehenden Ansprüche, des Weiteren umfassend Menthol.

8. Topische pharmazeutische Zusammensetzung nach Anspruch 7, bei der die Mentholmenge 0,10 bis 15,0 Gew.-% der Gesamtzusammensetzung beträgt, vorzugsweise bei 1,0 bis 10,0 Gew.-% der Gesamtzusammensetzung liegt.

9. Topische pharmazeutische Zusammensetzung nach einem der voranstehenden Ansprüche, des Weiteren umfassend Viskositätsverbesserer, Lösungsmittel, Konservierungsstoffe, Antioxidantien oder Mischungen daraus.

10. Topische pharmazeutische Zusammensetzung nach einem der voranstehenden Ansprüche, bei der sie in Form eines Gels, einer Salbe, einer Creme, eines Sprays oder einer Lotion vorliegt, wobei die bevorzugte Form die eines Gels ist.

11. Topische pharmazeutische Zusammensetzung nach einem der voranstehenden Ansprüche, folgendes umfassend
| | | |
|---|---|---|
| a. | Ketoprofen | 0,50 bis 15,0 Gew.-% |
| b. | Methylsulfonylmethan | 1,0 bis 20,0 Gew.-% |
| c. | Hydroxypropylcellulose | 0,05 bis 10,0 Gew.-% |
| d. | Carbomer | 0,01 bis 5,0 Gew.-% |
| e. | Dimethylsulfoxid | 0,5 bis 30,0 Gew.-% |
| f. | Polyethylenglykol | 1,0 bis 50,0 Gew.-% |
| g. | Menthol | 0,10 bis 15,0 Gew.-% |
| h. | Polysorbat | 0,05 bis 5,0 Gew.-% |
| i. | Propylparaben | 0,001 bis 2,0 Gew.-% |
| j. | Methylparaben | 0,01 bis 2,0 Gew.-% |
| k. | Butylhydroxytoluol | 0,001 bis 0,10 Gew.-% |
| l. | Glycerin | 1,0 bis 50,0 Gew.-% |
| m. | gereinigtes Wasser | 1,0 bis 50,0 Gew.-% |
| n. | Ethylalkohol | 1,0 bis 75,0 Gew.-% |
| o. | NaOH oder HCl | pH (5,5 ± 1,0) |

12. Verfahren zur Herstellung der topischen pharmazeutischen Zusammensetzung nach einem der voranstehenden Ansprüche, folgende Schritte umfassend
a. Zugabe von Carbomer, Glycerin, Polyethylenglykol, Dimethylsulfoxid in gereinigtes Wasser und Quellen des Gemisches unter Rühren über eine Zeitdauer von 60 Minuten und Homogenisieren zum Erhalt des ersten Gemisches,
b. Zugabe von NaOH oder HCI in das erste Gemisch zur Einstellung des pH-Wertes und weiteres Rühren,
c. Zugabe von Ethylalkohol in einen anderen Behälter und Zugabe von Ketoprofen, Methylsulfonylmethan, Menthol, Polysorbat, Methylparaben, Propylparaben, Butylhydroxytoluol und anschießend Zugabe von Hydroxypropylcellulose in dieses Gemisch und Rühren, bis sich alles aufgelöst hat, für eine Zeitdauer von ca. 90 Minuten, ungefähr 5 Minuten lang Homogenisieren zum Erhalt des zweiten Gemisches,
d. Zugabe des zweiten Gemisches in das erste Gemisch unter Rühren und anschließend Auffüllen des Volumens mit Ethylalkohol und weiteres Rühren für eine Zeitdauer von 10 Minuten,
e. Herbeiführen eines gelierten Zustands und Fortfahren mit dem Füllschritt.

13. Topische pharmazeutische Zusammensetzung nach einem der voranstehenden Ansprüche zur Verwendung bei der Behandlung von Schmerzen und Entzündungssymptomen im Zusammenhang mit dem Bewegungsapparat, Arthrose und rheumatoider Arthritis.

## Revendications

1. Composition pharmaceutique topique de kétoprofène et de méthylsulfonylméthane comprenant au moins un agent d'amélioration de pénétration et des agents gélifiants, dans laquelle l'agent d'amélioration de pénétration est du sulfoxyde diméthylique et les agents gélifiants sont de la cellulose hydroxypropylique et du carbomer, et dans laquelle le rapport pondéral de la cellulose hydroxypropylique et du carbomer est compris entre 20:1 et 1:10.

2. Composition pharmaceutique topique selon la revendication 1, dans laquelle le rapport pondéral de la cellulose hydroxypropylique et du carbomer est compris entre 5:1 et 1:5 du poids total de la composition.

3. Composition pharmaceutique topique selon la revendication 1, dans laquelle la quantité de sulfoxyde diméthylique est comprise entre 0,50 et 30,0 % du poids total de la composition, de préférence, elle est comprise entre 5,0 et 25,0 % du poids total de la composition.

4. Composition pharmaceutique topique selon l'une quelconque des revendications précédentes, comprenant en outre des agents tensio-actifs.

5. Composition pharmaceutique topique selon la revendication 4, dans laquelle l'agent tensio-actif est sélectionné parmi le groupe comprenant un polysorbate, du monostéarate glycérylique, du succinate de polyéthylène glycol, de l'acide oléique, de la diéthanolamine, du sulfate laurique de sodium, du glycol de propylène ou leurs mélanges ; de préférence, l'agent tensio-actif est un polysorbate.

6. Composition pharmaceutique topique selon la revendication 5, dans laquelle la quantité de polysorbate est comprise entre 0,05 et 5,0 % du poids total de la composition, de préférence, elle est comprise entre 0,10 et 3,0 % du poids total de la composition.

7. Composition pharmaceutique topique selon l'une quelconque des revendications précédentes, comprenant en outre du menthol.

8. Composition pharmaceutique topique selon la revendication 7, dans laquelle la quantité de menthol est comprise entre 0,10 et 15,0 % du poids total de la composition, de préférence, elle représente de 1,0 à 10,0 % du poids total de la composition.

9. Composition pharmaceutique topique selon l'une quelconque des revendications précédentes, comprenant en outre des agents d'amélioration de viscosité, des solvants de dissolution, des conservateurs, des antioxydants ou leurs mélanges.

10. Composition pharmaceutique topique selon l'une quelconque des revendications précédentes, dans laquelle elle est sous forme de gel, d'onguent, de crème, de produit pulvérisable ou de lotion, de préférence, elle est sous forme de gel.

11. Composition pharmaceutique topique selon l'une quelconque des revendications précédentes, comprenant :
| | |
|---|---|
| a. du kétoprofène | de 0,50 à 15,0% en poids |
| b. du méthylsulfonylméthane | de 1,0 à 20,0% en poids |
| c. de la cellulose hydroxypropylique | de 0,05 à 10,0 % en poids |
| d. du carbomer | de 0,01 à 5,0 % en poids |
| e. du sulfoxyde diméthylique | de 0,5 à 30,0 % en poids |
| f. du polyéthylène glycol | de 1,0 à 50,0 % en poids |
| g. du menthol | de 0,10 à 15,0 % en poids |
| h. un polysorbate | de 0,05 à 5,0 % en poids |
| i. du parabène propyllique | de 0,001 à 2,0 % en poids |
| j. du parabène méthylique | de 0,01 à 2,0 % en poids |
| k. de l'hydroxytoluène butylé | de 0,001 à 0,10 % en poids |
| I. de la glycérine | de 1,0 à 50,0 % en poids |
| m. de l'eau purifiée | de 1,0 à 50,0 % en poids |
| n. de l'alcool éthylique | de 1,0 à 75,0 % en poids |
| o. NaOH ou HCl | afin d'obtenir un pH de 5,5 ±1,0. |

12. Procédé de préparation de la composition pharmaceutique topique selon l'une quelconque des revendications précédentes, comprenant les étapes de :
a. ajout de carbomer, de glycérine, de polyéthylène glycol, de sulfoxyde diméthylique dans l'eau purifiée et épaississement de ce composé sous agitation pendant 60 mn, puis homogénéisation de manière à obtenir le premier composé,
b. ajout de NaOH ou de HCI au premier mélange, de manière à ajuster le pH, et agitation,
c. ajout d'alcool éthylique dans un autre conteneur et ajout de kétoprofène, de méthylsulfonylméthane, de menthol, de polysorbate, de parabène méthylique, de parabène propylique, d'hydroxytoluène butylé, puis ajout de cellulose hydroxypropylique dans ce composé et agitation jusqu'à ce qu'ils soient dissous pendant 90 mn environ, homogénéisation pendant 5 mn environ, de manière à obtenir le second composé,
d. ajout du second composé au premier composé sous agitation puis remplissage du volume avec de l'alcool éthylique et agitation pendant 10 mn supplémentaires.
e. obtention d'un état gélifié et poursuite par une étape de remplissage.

13. Composition pharmaceutique topique selon l'une quelconque des revendications précédentes, destinée à une utilisation dans le traitement de la douleur et des symptômes inflammatoires associés au système musculaire et squelettique, à l'ostéo-arthrite et à l'arthrite rhumatoïde.
